# EUROPEAN PATENT APPLICATION

(11) **EP 2 287 299 A1**
(43) Date of publication of application: **23.02.2011**
(21) Application number: 09762287.2
(22) Date of filing: 12.06.2009
(51) Int. Cl.: C12N 15/00, A23L 1/29, C12M 1/00, C12N 1/21, C12N 15/09

(54) **GENE THAT IMPARTS OXYGEN RESISTANCE AND APPLICATION THEREOF**

(30) Priority: 13.06.2008 JP 2008154845
(71) Applicant: Kabushiki Kaisha Yakult Honsha, Minato-ku Tokyo 1058860 (JP)
(72) Inventor: SERATA, Masaki, Tokyo 105-8660 (JP); SAKO, Tomoyuki, Tokyo 105-8660 (JP)
(74) Representative: Blodig, Wolfgang
(86) International application number: PCT/JP2009/002685
(87) International publication number: WO 2009/150856

(57) **Abstract**

To provide a gene useful for imparting oxygen resistance to a microorganism and use of the gene.

The oxygen-resistance-imparting gene encoding a protein selected from among the following proteins (a) to (c): (a) a protein having the amino acid sequence of SEQ ID NO: 2 or 6; (b) a protein which has an amino acid sequence equivalent to the amino acid sequence of (a), except that one to several amino acid residues are deleted, substituted, or added, and which exhibits oxygen-resistance-imparting activity; and (c) a protein which has an amino acid sequence having an identity of 85% or higher to the amino acid sequence of (a), and which exhibits oxygen-resistance-imparting activity.

SEQUENCE LISTING

<110>  KABUSHIKI KAISHA YAKULT HONSHA

<120>  Gene that imparts oxygen resistance and application thereof

<130>  YK0049

<150>  JP2008-154845
<151>  2008-06-13

<160>  8

<170>  PatentIn version 3.1

<210>  1
<211>  987
<212>  DNA
<213>  Lactobacillus casei YIT 9029

<220>
<221>  CDS
<222>  (1)..(987)
<223>

<400>  1
atg tca gtg cag cat gta tat gaa atc acg gta atc ggc ggc ggc ccg      48
Met Ser Val Gln His Val Tyr Glu Ile Thr Val Ile Gly Gly Gly Pro
1               5                   10                  15

gtt ggg atg ttc gcc gct ttt tat gcg ggt ttg cgc cag gca gac gtt      96
Val Gly Met Phe Ala Ala Phe Tyr Ala Gly Leu Arg Gln Ala Asp Val
            20                  25                  30

ttg tta ttg gag agt ctt gac gag ctt ggc ggc caa aca ggc aac tta      144
Leu Leu Leu Glu Ser Leu Asp Glu Leu Gly Gly Gln Thr Gly Asn Leu
        35                  40                  45

tac cct gac aaa att ctc tat gat att ggc gga ttc ccg cat gtt agt      192
Tyr Pro Asp Lys Ile Leu Tyr Asp Ile Gly Gly Phe Pro His Val Ser
    50                  55                  60

ggc aaa gac ctt gtc acg cag ctc aaa act cag ctc gtg cat ttt cat      240
Gly Lys Asp Leu Val Thr Gln Leu Lys Thr Gln Leu Val His Phe His
65                  70                  75                  80

cct gaa gtc aag acc gct acg gaa gta cag acg atc gat caa gat cca      288
Pro Glu Val Lys Thr Ala Thr Glu Val Gln Thr Ile Asp Gln Asp Pro
                85                  90                  95

gat ggc ttt ttc aca ttg cat aca agt caa ggc gat ttc cgc agt aaa      336
Asp Gly Phe Phe Thr Leu His Thr Ser Gln Gly Asp Phe Arg Ser Lys
            100                 105                 110

act gtg atc gtg gca acc ggt ggt ggt gct ttt acg ccg cgc aag tta      384
Thr Val Ile Val Ala Thr Gly Gly Gly Ala Phe Thr Pro Arg Lys Leu
        115                 120                 125

gct gtt gac tat gat ccg gca ttg gaa ggc aaa aaa ctg ttc tac ttc      432
Ala Val Asp Tyr Asp Pro Ala Leu Glu Gly Lys Lys Leu Phe Tyr Phe
    130                 135                 140

gtg cag gat ttg gaa act ttt cgg gat cag gaa gtg gcc gtg gct ggt      480
Val Gln Asp Leu Glu Thr Phe Arg Asp Gln Glu Val Ala Val Ala Gly
145                 150                 155                 160

ggt ggt gat tct gca att gat tgg gca ctg gca ctt gaa ccg gtc gcc      528
Gly Gly Asp Ser Ala Ile Asp Trp Ala Leu Ala Leu Glu Pro Val Ala
                165                 170                 175

aaa cat gtc agt ctg att cac cgt cgt gcc aaa ttt cgc ggc ttg gaa 576
Lys His Val Ser Leu Ile His Arg Arg Ala Lys Phe Arg Gly Leu Glu
            180                 185                 190

gcc agt gtt gct gca ctt gaa aaa agc agt gtg aca att cag acg cct 624
Ala Ser Val Ala Ala Leu Glu Lys Ser Ser Val Thr Ile Gln Thr Pro
        195                 200                 205

tac ctg atc gaa tca gtc acc cct caa gat gat cag tta gcg att aag 672
Tyr Leu Ile Glu Ser Val Thr Pro Gln Asp Asp Gln Leu Ala Ile Lys
    210                 215                 220

ttg aag gaa gtt cgc ggc gag gca cag cca acg ctc acc gtc gac aag 720
Leu Lys Glu Val Arg Gly Glu Ala Gln Pro Thr Leu Thr Val Asp Lys
225                 230                 235                 240

ctg ttg atc aat tat gga ttt gtc acc gac aaa cac cac cta cag gct 768
Leu Leu Ile Asn Tyr Gly Phe Val Thr Asp Lys His His Leu Gln Ala
                245                 250                 255

tgg caa ctt gac acg gat cgt aat ggt ata ctg gtg gac aca caa atg 816
Trp Gln Leu Asp Thr Asp Arg Asn Gly Ile Leu Val Asp Thr Gln Met
            260                 265                 270

cag acg agt cgt ccc ggc att ttt gca att ggc gac gct gtc agc tat 864
Gln Thr Ser Arg Pro Gly Ile Phe Ala Ile Gly Asp Ala Val Ser Tyr
        275                 280                 285

gct ggc aag ttg ccg ttg att gcc agc ggt ttt gga gaa gcg cca acc 912
Ala Gly Lys Leu Pro Leu Ile Ala Ser Gly Phe Gly Glu Ala Pro Thr
    290                 295                 300

gct atc aat gag gcc tta agc cgc ttg tat cca gat cga cgt cag gcc 960
Ala Ile Asn Glu Ala Leu Ser Arg Leu Tyr Pro Asp Arg Arg Gln Ala
305                 310                 315                 320

ttg cac agt act cag ttg tat cat taa 987
Leu His Ser Thr Gln Leu Tyr His
                325

<210> 2
<211> 328
<212> PRT
<213> Lactobacillus casei YIT 9029

<400> 2

Met Ser Val Gln His Val Tyr Glu Ile Thr Val Ile Gly Gly Gly Pro
1               5                   10                  15

Val Gly Met Phe Ala Ala Phe Tyr Ala Gly Leu Arg Gln Ala Asp Val
            20                  25                  30

Leu Leu Leu Glu Ser Leu Asp Glu Leu Gly Gly Gln Thr Gly Asn Leu
        35                  40                  45

Tyr Pro Asp Lys Ile Leu Tyr Asp Ile Gly Gly Phe Pro His Val Ser
    50                  55                  60

Gly Lys Asp Leu Val Thr Gln Leu Lys Thr Gln Leu Val His Phe His
65                  70                  75                  80

Pro Glu Val Lys Thr Ala Thr Glu Val Gln Thr Ile Asp Gln Asp Pro
                85                  90                  95

Asp Gly Phe Phe Thr Leu His Thr Ser Gln Gly Asp Phe Arg Ser Lys
            100                 105                 110

Thr Val Ile Val Ala Thr Gly Gly Gly Ala Phe Thr Pro Arg Lys Leu
        115                 120                 125

Ala Val Asp Tyr Asp Pro Ala Leu Glu Gly Lys Lys Leu Phe Tyr Phe
    130                 135                 140

Val Gln Asp Leu Glu Thr Phe Arg Asp Gln Glu Val Ala Val Ala Gly
145                 150                 155                 160

Gly Gly Asp Ser Ala Ile Asp Trp Ala Leu Ala Leu Glu Pro Val Ala
                165                 170                 175

Lys His Val Ser Leu Ile His Arg Arg Ala Lys Phe Arg Gly Leu Glu
            180                 185                 190

Ala Ser Val Ala Ala Leu Glu Lys Ser Ser Val Thr Ile Gln Thr Pro
        195                 200                 205

Tyr Leu Ile Glu Ser Val Thr Pro Gln Asp Asp Gln Leu Ala Ile Lys
    210                 215                 220

Leu Lys Glu Val Arg Gly Glu Ala Gln Pro Thr Leu Thr Val Asp Lys
225                 230                 235                 240

Leu Leu Ile Asn Tyr Gly Phe Val Thr Asp Lys His His Leu Gln Ala
                245                 250                 255

Trp Gln Leu Asp Thr Asp Arg Asn Gly Ile Leu Val Asp Thr Gln Met
            260                 265                 270

Gln Thr Ser Arg Pro Gly Ile Phe Ala Ile Gly Asp Ala Val Ser Tyr
        275                 280                 285

Ala Gly Lys Leu Pro Leu Ile Ala Ser Gly Phe Gly Glu Ala Pro Thr
    290                 295                 300

Ala Ile Asn Glu Ala Leu Ser Arg Leu Tyr Pro Asp Arg Arg Gln Ala
305                 310                 315                 320

Leu His Ser Thr Gln Leu Tyr His
                325

<210>  3
<211>  26
<212>  DNA
<213>  Artificial Sequence

<220>
<223>   primer sequence specific to fnr

<400>  3
cgggatccag atggcttttt cacatt                                          26


<210>  4
<211>  28
<212>  DNA
<213>  Artificial Sequence

<220>
<223>  primer sequence specific to fnr

<400>  4
aaactgcagc caccagtata ccattacg                                        28


<210>  5
<211>  987
<212>  DNA
<213>  Lactobacillus rhamnosus ATCC 53103

<220>
<221>  CDS
<222>  (1)..(987)
<223>

<400>  5
atg tca gtg cag cat gta tat gaa atc act gtg att ggc ggc ggc ccg       48
Met Ser Val Gln His Val Tyr Glu Ile Thr Val Ile Gly Gly Gly Pro
1               5                   10                  15

gtc ggc atg ttc gcc gct ttt tat gcc ggg cta cgt caa gcg gat gtc       96
Val Gly Met Phe Ala Ala Phe Tyr Ala Gly Leu Arg Gln Ala Asp Val
            20                  25                  30

tta tta tta gaa agt ctt gat gaa ctc ggt ggt caa acc gga aat tta      144
Leu Leu Leu Glu Ser Leu Asp Glu Leu Gly Gly Gln Thr Gly Asn Leu
        35                  40                  45

tat ccg gcc aaa att ctt tat gat atc ggc ggc ttc cct cat gtc agc      192
Tyr Pro Ala Lys Ile Leu Tyr Asp Ile Gly Gly Phe Pro His Val Ser
    50                  55                  60

ggt aag gat ttg gtc gct caa ctg aag act cag ctg atg cat ttt aaa      240
Gly Lys Asp Leu Val Ala Gln Leu Lys Thr Gln Leu Met His Phe Lys
65                  70                  75                  80

ccg gac gtc aaa aca gct act gaa gtt cag aca att gac gag gct gat      288
Pro Asp Val Lys Thr Ala Thr Glu Val Gln Thr Ile Asp Glu Ala Asp
                85                  90                  95

gat ggc act ttt att ctg cat acg agc caa gga gat ttc cac act aaa      336
Asp Gly Thr Phe Ile Leu His Thr Ser Gln Gly Asp Phe His Thr Lys
            100                 105                 110

acg gtc att gtt gcg act ggt ggc ggt gcc ttc acg ccg cgt aaa tta      384
Thr Val Ile Val Ala Thr Gly Gly Gly Ala Phe Thr Pro Arg Lys Leu
        115                 120                 125

gcc gtt gat tat gac ccc gct tta gaa ggt cag caa ctt ttt tat ttt      432
Ala Val Asp Tyr Asp Pro Ala Leu Glu Gly Gln Gln Leu Phe Tyr Phe
    130                 135                 140

gtt caa gat ctg gag act ttt cgc gat cag gaa gtt gcc atc gct ggc      480
Val Gln Asp Leu Glu Thr Phe Arg Asp Gln Glu Val Ala Ile Ala Gly
145                 150                 155                 160

ggc ggc gat tct gcc att gat tgg gct ttg gcg cta gag ccg att gcc 528
Gly Gly Asp Ser Ala Ile Asp Trp Ala Leu Ala Leu Glu Pro Ile Ala
                165                 170                 175

cgg cat gtt act tta att cat cgc cgc tcc aaa ttt cgc gga ctt gag 576
Arg His Val Thr Leu Ile His Arg Arg Ser Lys Phe Arg Gly Leu Glu
            180                 185                 190

gcg agt gtg gct gca ctt gaa aag agt agt gtg acg atc gaa acc ccg 624
Ala Ser Val Ala Ala Leu Glu Lys Ser Ser Val Thr Ile Glu Thr Pro
        195                 200                 205

tat tta atc gaa gcc gtc acg cac gaa aat aag cgc ctg gct gta cgc 672
Tyr Leu Ile Glu Ala Val Thr His Glu Asn Lys Arg Leu Ala Val Arg
    210                 215                 220

tta aaa gaa gtt cgt ggt gag gac cag ccc acc tta aca gtc gac aaa 720
Leu Lys Glu Val Arg Gly Glu Asp Gln Pro Thr Leu Thr Val Asp Lys
225                 230                 235                 240

ttg ctg gtt aat tat ggc ttt gtt act gac aaa cgg cat ctt caa gca 768
Leu Leu Val Asn Tyr Gly Phe Val Thr Asp Lys Arg His Leu Gln Ala
                245                 250                 255

tgg cag ttc aaa acc gat cgt aat ggc atc ctg gtt gat tca cag atg 816
Trp Gln Phe Lys Thr Asp Arg Asn Gly Ile Leu Val Asp Ser Gln Met
            260                 265                 270

caa acc agc cga ccc gga att ttt gcg att ggt gat gcc gtc agt tat 864
Gln Thr Ser Arg Pro Gly Ile Phe Ala Ile Gly Asp Ala Val Ser Tyr
        275                 280                 285

gcc gga aag ttg cca ttg att gcc agc ggc ttt ggt gag gca ccg act 912
Ala Gly Lys Leu Pro Leu Ile Ala Ser Gly Phe Gly Glu Ala Pro Thr
    290                 295                 300

gcc att aac gaa gcg ctc agt cgg ctt tac ccg gaa cgc cgt caa gct 960
Ala Ile Asn Glu Ala Leu Ser Arg Leu Tyr Pro Glu Arg Arg Gln Ala
305                 310                 315                 320

ttg cac agt acc caa ctt tat cga taa 987
Leu His Ser Thr Gln Leu Tyr Arg
                325

<210> 6
<211> 328
<212> PRT
<213> Lactobacillus rhamnosus ATCC 53103

<400> 6

Met Ser Val Gln His Val Tyr Glu Ile Thr Val Ile Gly Gly Gly Pro
1               5                   10                  15

Val Gly Met Phe Ala Ala Phe Tyr Ala Gly Leu Arg Gln Ala Asp Val
            20                  25                  30

Leu Leu Leu Glu Ser Leu Asp Glu Leu Gly Gly Gln Thr Gly Asn Leu
        35                  40                  45

Tyr Pro Ala Lys Ile Leu Tyr Asp Ile Gly Gly Phe Pro His Val Ser
    50                  55                  60

Gly Lys Asp Leu Val Ala Gln Leu Lys Thr Gln Leu Met His Phe Lys
65                  70                  75                  80

Pro Asp Val Lys Thr Ala Thr Glu Val Gln Thr Ile Asp Glu Ala Asp
                85                  90                  95

Asp Gly Thr Phe Ile Leu His Thr Ser Gln Gly Asp Phe His Thr Lys
            100                 105                 110

Thr Val Ile Val Ala Thr Gly Gly Gly Ala Phe Thr Pro Arg Lys Leu
        115                 120                 125

Ala Val Asp Tyr Asp Pro Ala Leu Glu Gly Gln Gln Leu Phe Tyr Phe
    130                 135                 140

Val Gln Asp Leu Glu Thr Phe Arg Asp Gln Glu Val Ala Ile Ala Gly
145                 150                 155                 160

Gly Gly Asp Ser Ala Ile Asp Trp Ala Leu Ala Leu Glu Pro Ile Ala
                165                 170                 175

Arg His Val Thr Leu Ile His Arg Arg Ser Lys Phe Arg Gly Leu Glu
            180                 185                 190

Ala Ser Val Ala Ala Leu Glu Lys Ser Ser Val Thr Ile Glu Thr Pro
        195                 200                 205

Tyr Leu Ile Glu Ala Val Thr His Glu Asn Lys Arg Leu Ala Val Arg
    210                 215                 220

Leu Lys Glu Val Arg Gly Glu Asp Gln Pro Thr Leu Thr Val Asp Lys
225                 230                 235                 240

Leu Leu Val Asn Tyr Gly Phe Val Thr Asp Lys Arg His Leu Gln Ala
                245                 250                 255

Trp Gln Phe Lys Thr Asp Arg Asn Gly Ile Leu Val Asp Ser Gln Met
            260                 265                 270

Gln Thr Ser Arg Pro Gly Ile Phe Ala Ile Gly Asp Ala Val Ser Tyr
        275                 280                 285

Ala Gly Lys Leu Pro Leu Ile Ala Ser Gly Phe Gly Glu Ala Pro Thr
    290                 295                 300

Ala Ile Asn Glu Ala Leu Ser Arg Leu Tyr Pro Glu Arg Arg Gln Ala
305                 310                 315                 320

Leu His Ser Thr Gln Leu Tyr Arg
                325

<210>  7

<211>  25
<212>  DNA
<213>  Artificial Sequence

<220>
<223>  primer sequence specific to fnr-r

<400>  7
tatggatcca cactaaaacg gtcat                                          25


<210>  8
<211>  22
<212>  DNA
<213>  Artificial Sequence

<220>
<223>  primer sequence specific to fnr-r

<400>  8
attctgcagt cggtgcctca cc                                             22

## Description

### Technical Field

The present invention relates to a gene which imparts oxygen resistance to a microorganism, and to use of the gene.

### Background Art

Many aerobic microorganisms have a respiratory chain responsible for oxygen metabolism, and can acquire energy in the presence of oxygen, and can be grown well in the presence of oxygen. Such aerobic microorganisms also have a mechanism of detoxifying superoxide anion or hydrogen peroxide-which may be generated from a portion of oxygen through metabolism thereof-with the aid of, for example, superoxide dismutase, catalase, or peroxidase, which enzyme eliminates the toxicity of such a reactive oxygen species.

Lactic acid bacteria, which are known to be useful among anaerobic microorganisms, are facultative anaerobes, and are considered to have neither a respiratory chain nor catalase. However, many lactic acid bacteria can be grown even in the presence of oxygen, and exhibit oxygen resistance. Hitherto, some studies have been conducted on the oxygen resistance mechanism of lactic acid bacteria, and have shown that lactic acid bacteria have, for example, NADH oxidase or pyruvate oxidase as an oxygen-metabolizing enzyme. As has been reported, NADH oxidase is classified into two types (i.e., water-generating type and hydrogen peroxide-generating type), and water-generating NADH oxidase detoxifies oxygen by four-electron reduction to form water, whereas hydrogen peroxide-generating NADH oxidase generates hydrogen peroxide by two-electron reduction. As has also been reported, in a lactic acid bacterium such as *Streptococcus mutans,* alkyl hydroperoxide reductase converts hydrogen peroxide into water, and these enzymes function as a two-component peroxidase.
Some lactic acid bacteria have been reported to have, for example, superoxide dismutase, catalase, or NADH peroxidase, which eliminates superoxide anion or hydrogen peroxide generated from oxygen.

Studies have suggested that *Lactobacillus plantarum* WCFS1 exhibits enhanced resistance to oxidative stress (e.g., hydrogen peroxide) by enhancing expression of the thioredoxin reductase gene, and thus, in *Lactobacillus plantarum* WCFS1, thioredoxin reductase plays an important role in resistance to oxidative stress (Non-Patent Document 1).
In *Bacteroides fragilis,* which is an anaerobic Gram-negative bacterium, only a thioredoxin-thioredoxin reductase system is considered to be responsible for oxidation-reduction reaction of thiol/disulfide. It has been reported that when thioredoxin reductase is deleted, the bacterium cannot be grown even under anaerobic conditions without addition of a reducing agent (e.g., cysteine or dithiothreitol) (Non-Patent Document 2).
As has been reported, *Escherichia coli* includes therein a glutathione-glutathione reductase system and a thioredoxin-thioredoxin reductase system, which is essential for maintaining the intracellular environment in a reduced state, and gene-disrupted strains involved in such a system are sensitive to oxidative stress (e.g., hydrogen peroxide).

As has been reported, growth of *Lactococcus lactis* is inhibited under aerobic conditions through disruption of thioredoxin reductase (Non-Patent Document 3). However, relation between growth inhibition of the bacterium and oxygen resistance thereof has not been elucidated, since growth of the bacterium under aerobic conditions is restored by addition of dithiothreitol, and the amount of cells of the bacterium after 24-hour culturing is nearly equal to that of wild-type cells of the bacterium.

As has also been reported, a mutant strain of *Streptococcus mutans* obtained through knockout of both NADH oxidase and alkyl hydroperoxide reductase (ahpC) exhibits oxygen resistance, and thus the gene for another iron-binding protein is responsible for oxygen resistance (Patent Document 1). However, such a gene is not necessarily present in all microorganisms, and the mechanism of oxygen resistance has not yet been fully elucidated. As described above, a plurality of genes are responsible for oxygen resistance; i.e., it is not the case that only a single gene is responsible for imparting oxygen resistance to a microorganism. Therefore, difficulty is encountered in practically using such a gene for imparting oxygen resistance to a microorganism.

It has been known that *Lactobacillus casei* YIT 9029 (FERM BP-1366) and *Lactobacillus rhamnosus* ATCC 53103 can be well grown under aerobic conditions, and they exhibit various physiological effects on humans. However, a gene responsible for oxygen resistance has not yet been identified in these lactic acid bacteria.

### Related Art Document

### Patent Document

Patent Document 1: JP-A-2001-32792

### Non-Patent Document

Non-Patent Document 1: L. Mariela Serrano et al., Microbial Cell Factories 6: 29 (2007)
Non-Patent Document 2: Edson R., Rocha et al., J. Bacteriol. 189: 8015-8023 (2007)
Non-Patent Document 3: Karin Vido et al., J. Bacteriol. 187: 601-610 (2005)

### Summary of the Invention

### Problems to be Solved by the Invention

The present invention relates to provision of a gene which imparts oxygen resistance to a microorganism (hereinafter the gene may be referred to as an "oxygen-resistance-imparting gene"), as well as use of the gene. Means for Solving the Problems

The present inventors have conducted studies on the genomic information of *Lactobacillus casei* YIT 9029 and *Lactobacillus rhamnosus* ATCC 53103 and prepared gene-disrupted strains thereof, and as a result have found that there is a gene essential for oxygen resistance, and when such a gene is introduced into a microorganism, oxygen resistance can be imparted to or enhanced in the microorganism.

Accordingly, the present invention provides the following.
1) An oxygen-resistance-imparting gene encoding a protein selected from among the following proteins (a) to (c):
   (a) a protein having the amino acid sequence of SEQ ID NO: 2 or 6;
   (b) a protein which has an amino acid sequence equivalent to the amino acid sequence of (a), except that one to several amino acid residues are deleted, substituted, or added, and which exhibits oxygen-resistance-imparting activity; and
   (c) a protein which has an amino acid sequence having an identity of 85% or higher to the amino acid sequence of (a), and which exhibits oxygen-resistance-imparting activity.
2) An oxygen-resistance-imparting gene having a polynucleotide selected from among the following polynucleotides (d) to (f):
   (d) a polynucleotide having the nucleotide sequence of SEQ ID NO: 1 or 5;
   (e) a polynucleotide which hybridizes, under stringent conditions, with a polynucleotide having a nucleotide sequence complementary to the nucleotide sequence of (d), and which encodes a protein exhibiting oxygen-resistance-imparting activity; and
   (f) a polynucleotide which has a nucleotide sequence having an identity of 75% or higher to the nucleotide sequence of (d), and which encodes a protein exhibiting oxygen-resistance-imparting activity.
3) A method for imparting oxygen resistance to or enhancing oxygen resistance in a microorganism, comprising introducing any of the aforementioned genes into the microorganism, or modifying the gene present in the microorganism.
4) A microorganism into which any of the aforementioned genes has been introduced or in which the gene has been modified.
5) A food or beverage containing the aforementioned microorganism.
6) A drug containing the aforementioned microorganism.
7) A screening method for selecting a microorganism exhibiting oxygen resistance, comprising determining the presence or absence of any of the aforementioned genes, and/or determining the level of expression of the gene.
8) A recombinant vector containing any of the aforementioned polynucleotides or a portion thereof.
9) A host microorganism containing the aforementioned recombinant vector.
10) A nucleic acid fragment which specifically hybridizes with any of the aforementioned polynucleotides.
11) A DNA array or DNA chip containing any of the aforementioned polynucleotides or a portion thereof.

### Effects of the Invention

Employment of the gene or polynucleotide of the present invention can impart oxygen resistance to or enhance oxygen resistance in a microorganism, or realizes selection, through screening, of a microorganism exhibiting oxygen resistance.
Employment of a microorganism into which the gene of the present invention has been introduced or in which the gene has been modified realizes production of a food, beverage, or drug exhibiting oxygen resistance of interest. Since the number of living cells of the microorganism can be maintained at a high level even in the presence of oxygen, production of such a product does not require an anaerobic apparatus or an anaerobic storage container; i.e., the product can be produced at low cost. In general, the greater the number of living cells of a microorganism, the higher the physiological effects of the microorganism. Thus, the present invention enables a microorganism to effectively exhibit its physiological effects.

### Brief Description of the Drawings

[Fig. 1]

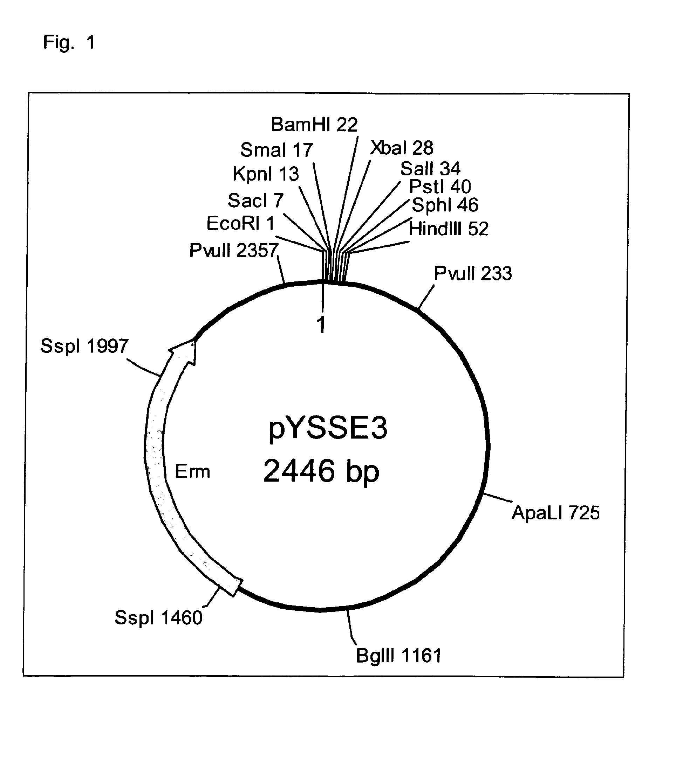

   Fig. 1 shows pYSSE3.
[Fig. 2]

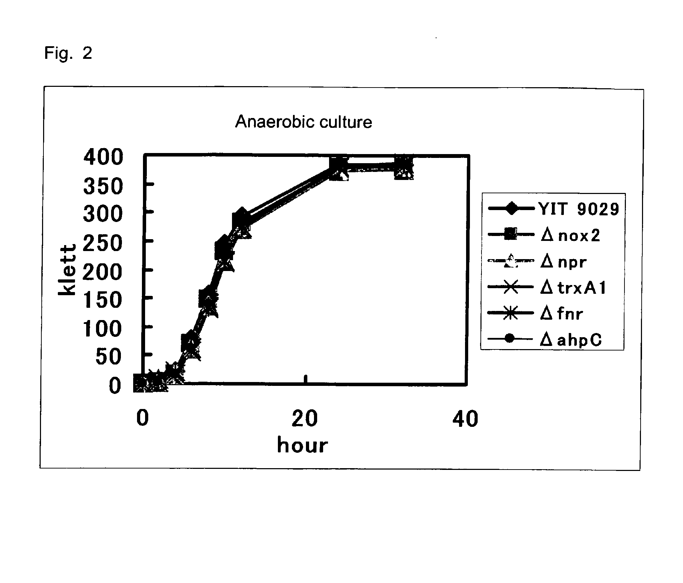

   Fig. 2 shows growth, under anaerobic culture conditions, of an *fnr* gene-disrupted strain, as well as strains in which known oxygen-resistance-related genes have been disrupted.
[Fig. 3]

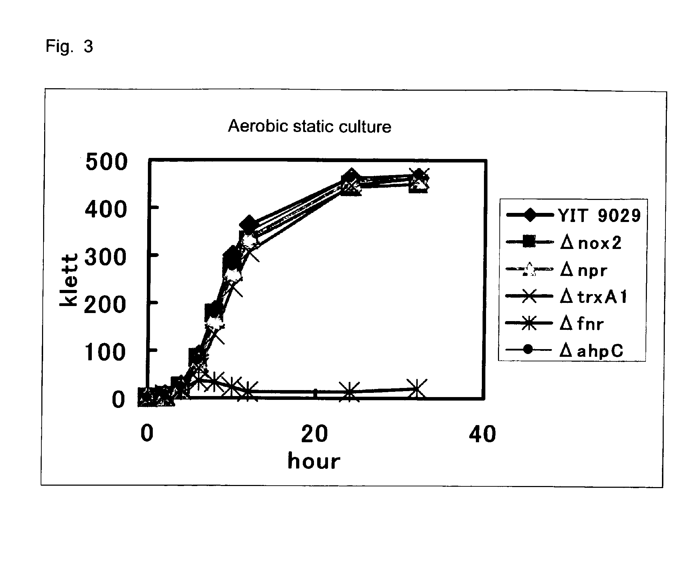

   Fig. 3 shows growth, under aerobic static culture conditions, of an *fnr* gene-disrupted strain, as well as strains in which known oxygen-resistance-related genes have been disrupted.
[Fig. 4]

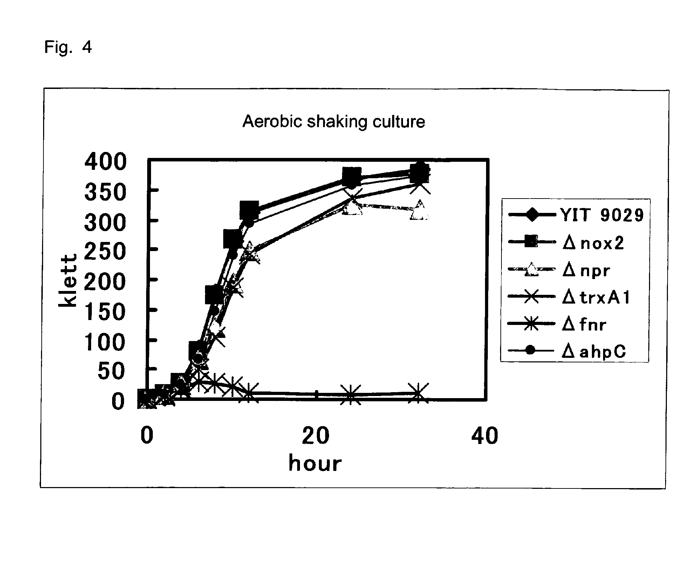

   Fig. 4 shows growth, under aerobic shaking culture conditions, of an *fnr* gene-disrupted strain, as well as strains in which known oxygen-resistance-related genes have been disrupted.

### Best Modes for Carrying Out the Invention

In the present invention, identity (homology) between amino acid sequences and that between nucleotide sequences can be determined through the Lipman-Pearson method (Lipman, D. J. and Pearson, W. R. 1985. Rapid and sensitive protein similarity searches. Science 227: 1435-1441) by use of genetic information processing software GENETYX (product of Genetyx Corporation) employing an identity analysis (search homology) program. Specifically, homology (%) is calculated through, for example, analysis of data on comparison between a known gene and a gene of *Lactobacillus casei* YIT 9029 or *Lactobacillus rhamnosus* ATCC 53103 (parameters are as follows: unit size to compare = 2, pick up location = 5).

As used herein, the term "gene" refers to a double-stranded DNA fragment, as well as a single-stranded DNA fragment (e.g., a sense-strand or antisense-strand fragment) which forms such a double-stranded DNA fragment. No particular limitation is imposed on the length of such a DNA fragment. Examples of the polynucleotide include RNA and DNA fragments, and examples of DNA fragments include cDNA, genomic DNA, and synthetic DNA fragments.

The oxygen-resistance-imparting gene of the present invention is a gene found in *Lactobacillus casei* YIT 9029 or *Lactobacillus rhamnosus* ATCC 53103 and named *fnr (Lactobacillus casei fnr* or *Lactobacillus rhamnosus fnr* (may be referred to as *"fnr-r"*)), or a gene deduced from the *fnr* gene. The gene of the present invention encodes a protein exhibiting oxygen-resistance-imparting activity.

Specifically, the oxygen-resistance-imparting gene of the present invention is a gene encoding a protein selected from among the following proteins (a) to (c):
(a) a protein having the amino acid sequence of SEQ ID NO: 2 or 6;
(b) a protein which has an amino acid sequence equivalent to the amino acid sequence of (a), except that one to several amino acid residues are deleted, substituted, or added, and which exhibits oxygen-resistance-imparting activity; and
(c) a protein which has an amino acid sequence having an identity of 85% or higher to the amino acid sequence of (a), and which exhibits oxygen-resistance-imparting activity.

The protein having the amino acid sequence of SEQ ID NO: 2 is a protein derived from *Lactobacillus casei* YIT 9029, and the protein having the amino acid sequence of SEQ ID NO: 6 is a protein derived from *Lactobacillus rhamnosus* ATCC 53103.
The amino acid sequence of SEQ ID NO: 2 or 6 in which one or more amino acid residues are deleted, substituted, or added encompasses an amino acid sequence obtained through deletion, substitution, or addition of one to several amino acid residues (preferably 1 to 10 amino acid residues). As used herein, "addition" encompasses addition of one to several amino acid residues to both ends of an amino acid sequence.

As used herein, "deletion, substitution, or addition of an amino acid residue(s)" encompasses deletion, substitution, or addition of an amino acid residue(s) in a protein having an amino acid sequence of, for example, SEQ ID NO: 2, resulting from, for example, naturally occurring mutation (e.g., single nucleotide substitution) or artificial mutation (e.g., site-directed mutagenesis or mutagenic treatment). In the case of artificial deletion, substitution, or addition of an amino acid residue(s), for example, a polynucleotide having a nucleotide sequence encoding an amino acid sequence of, for example, SEQ ID NO: 2 is subjected to a conventional site-directed mutagenesis, followed by expression of the polynucleotide through a customary method.
Amino acid residue substitution may be, for example, substitution by an amino acid residue exhibiting properties (e.g., hydrophobicity, electric charge, pK, and conformational feature) similar to those of the original amino acid residue.

The expression "amino acid sequence having an identity of 85% or higher to the amino acid sequence of (a)" refers to an amino acid sequence which, upon appropriate alignment, exhibits an identity of 85% or higher (preferably 90% or higher, more preferably 95% or higher) to the amino acid sequence of SEQ ID NO: 2 or 6.
The protein having the amino acid sequence of SEQ ID NO: 2 has an identity of 89.0% in amino acid sequence to the protein having the amino acid sequence of SEQ ID NO: 6.

As used herein, the expression "impart oxygen resistance" refers to the case where oxygen resistance is imparted to a microorganism; specifically, a microorganism is rendered capable of growing even in the presence of oxygen, and/or a grown microorganism is not killed even in the presence of oxygen. Also, the expression "impart oxygen resistance" encompasses the case where the oxygen-resistance-imparted microorganism has the ability to eliminate oxygen, the ability to eliminate reactive oxygen species generated from oxygen, the ability to change the intracellular environment from an oxidative state to a reducing state, or the ability to reduce DNA, protein, lipid, etc. which have been oxidized by oxygen or reactive oxygen species.
Specifically, the expression "impart oxygen resistance" refers to the case where when the oxygen-resistance-imparted microorganism is cultured on an agar plate medium in air, the microorganism can form colonies, or the case where the microorganism can be grown in a liquid medium in which dissolved oxygen is saturated by shaking, or in which oxygen contained in air is dissolved naturally.

Next will be described the homology (identity) between known proteins and a protein having the amino acid sequence of SEQ ID NO: 2 or 6. For homology search, by use of genetic information processing software GENETYX, the amino acid sequence of a protein defined by any of the aforementioned nucleotide sequences was compared with the amino acid sequence of a protein defined generally by a disclosed nucleotide sequence of the genome or a DNA fragment of lactic acid bacteria, to thereby search for genes having homology. When the entire amino acid sequence (or a portion thereof having a maximum possible length) of a protein has a homology of 50% or higher to that of the corresponding protein, these proteins are regarded as having homology.

The protein having the amino acid sequence of SEQ ID NO: 2 (FNR) has an identity of 99.4% in amino acid sequence to a protein encoded by *trxB* annotated as encoding thioredoxin reductase derived from *Lactobacillus casei* ATCC 334 strain. The protein having the amino acid sequence of SEQ ID NO: 6 (FNR-R) has an identity of 89.3% in amino acid sequence to the protein encoded by *trxB.*

The oxygen-resistance-imparting gene of the present invention is preferably a gene having a polynucleotide selected from among the following polynucleotides (d) to (f):
(d) a polynucleotide having the nucleotide sequence of SEQ ID NO: 1 or 5;
(e) a polynucleotide which hybridizes, under stringent conditions, with a polynucleotide having a nucleotide sequence complementary to the nucleotide sequence of (d), and which encodes a protein exhibiting oxygen-resistance-imparting activity; and
(f) a polynucleotide which has a nucleotide sequence having an identity of 75% or higher to the nucleotide sequence of (d), and which encodes a protein exhibiting oxygen-resistance-imparting activity.

The polynucleotide having the nucleotide sequence of SEQ ID NO: 1 is a DNA fragment derived from *Lactobacillus casei* YIT 9029 (*Lactobacillus casei fnr* gene), and the polynucleotide having the nucleotide sequence of SEQ ID NO: 5 is a DNA fragment derived from *Lactobacillus rhamnosus* ATCC 53103 (*Lactobacillus rhamnosus fnr* gene (*fnr-r* gene)). Each of these polynucleotides imparts oxygen resistance to a microorganism, and enables the microorganism to grow in the presence of oxygen.

As used herein, the expression "under stringent conditions" refers to, for example, the case where hybridization is carried out under conditions described in Molecular Cloning - a Laboratory manual 2nd edition (Sambrook, et al., 1989); specifically, the case where hybridization is carried out in a solution containing 6 x SSC (composition of 1 x SSC: 0.15 M sodium chloride, 0.015 M sodium citrate, pH 7.0), 0.5% SDS, 5 x Denhardt's solution, and 100 mg/mL herring sperm DNA together with a polynucleotide having a nucleotide sequence complementary to any of the aforementioned nucleotide sequences constantly at 65°C for 8 to 16 hours.

The expression "nucleotide sequence having an identity of 75% or higher to the nucleotide sequence of (d)" refers to a nucleotide sequence which, upon appropriate alignment, exhibits an identity of 75% or higher (preferably 90% or higher, more preferably 95% or higher) to the nucleotide sequence of SEQ ID NO: 1 or 5.
The polynucleotide having the nucleotide sequence of SEQ ID NO: 1 has an identity of 75.3% in nucleotide sequence to the polynucleotide having the nucleotide sequence of SEQ ID NO: 5.

The gene of the present invention can be readily obtained through a customary PCR technique by using a primer set prepared on the basis of the nucleotide sequence of SEQ ID NO: 1 or 5, and using, as a template, DNA of *Lactobacillus casei* YIT 9029 or *Lactobacillus rhamnosus* ATCC 53103.
Specifically, the gene of the present invention can be obtained through, for example, PCR by using a set of chemically synthesized oligonucleotides A and B (oligonucleotide A has a sequence including the N-terminal start codon of any of the aforementioned genes, and oligonucleotide B has a sequence complementary to a sequence including the stop codon of the gene), and using, as a template, DNA of *Lactobacillus casei* YIT 9029. For effective cloning of the thus-obtained gene fragment into, for example, a plasmid vector, a sequence for restriction enzyme cleavage may be added on the 5'-end side of the oligonucleotide primer. The primer which may be employed in the present invention is generally, for example, a nucleotide chemically synthesized on the basis of information on the nucleotide sequence of the gene of the present invention, and may be the gene of the present invention which has already been obtained or a fragment thereof. Such a nucleotide has a partial nucleotide sequence corresponding to, for example, SEQ ID NO: 1 or 5, and includes, for example, 10 to 50 consecutive nucleotides (preferably 15 to 35 consecutive nucleotides).
When, for example, a DNA fragment having a length of 2,000 base pairs is prepared, PCR is carried out under the following conditions: 94°C for 2 minutes, (95°C for 10 seconds, 52°C for 10 seconds, 72°C for 2 minutes) x 30 cycles, and 72°C for 7 minutes.

The gene of the present invention may be artificially synthesized by means of a DNA synthesizer on the basis of the corresponding nucleotide sequence.

The gene of the present invention is a gene responsible for imparting oxygen resistance. Therefore, when the gene of the present invention is introduced into a microorganism, or when the gene present in the microorganism is modified, oxygen resistance can be imparted to the microorganism, or oxygen resistance of the microorganism can be enhanced.
The gene of the present invention may be introduced into a microorganism which does not originally have the gene. Introduction of the gene may be carried out through, for example, the competence method using DNA uptake ability, the protoplast PEG method using a protoplast, or electroporation using high-voltage pulses. Particularly, electroporation is preferably employed. Integration of the gene into the chromosome of a microorganism may be carried out through homologous recombination or site-specific integration.

Modification of the gene of the present invention may be enhancement of expression of the gene.
Enhancement of expression of the gene of the present invention may be carried out through, for example, a method in which a recombinant plasmid carrying the gene is introduced into a microorganism of interest; a method in which the gene is integrated into another site of the chromosome through site-specific recombination, to thereby increase the number of copies of the gene in a microorganism; or a method in which the level of expression of the gene is increased by modifying a region for controlling expression of the gene or by modifying a regulatory gene. Particularly preferred is a method of increasing the number of copies of the gene. Specifically, the number of copies of the gene of interest may be increased in microbial cells through the following procedure: the gene (including the original promoter sequence and ribosome-binding site of the gene) or the polynucleotide (prepared by ligating only a polypeptide-encoding region of the gene to the downstream of a promoter and a ribosome-binding site which have been separated from another gene or chemically synthesized) is cloned into a plasmid having a plurality copies per microbial cell, and the plasmid is introduced into microbial cells through electroporation or a similar technique.

In the case of a microorganism which originally has the gene of the present invention, expression of the gene may be inhibited or suppressed, to thereby reduce oxygen resistance of the microorganism.
For inhibition of expression of the gene of the present invention, the gene may be disrupted or deleted through the insertion-inactivation method in which a DNA fragment entirely different from a target gene is inserted into the gene, or the stepwise double crossover method in which the entirety or a portion of a target gene is deleted by stepwise homologous recombination. Particularly, the stepwise double crossover method is preferably employed.
Specifically, when the entirety or a portion of a target gene is deleted, two regions sandwiching the deletion region are separated from chromosomal DNA or separated following amplification by PCR, and the two DNA fragments are cloned into a plasmid vector (e.g., pYSSE3) which can replicate in *Escherichia coli* but cannot in a microorganism of interest, so that the fragments are aligned in the same direction as the original direction. Subsequently, the resultant recombinant plasmid DNA is introduced, through electroporation or a similar technique, into a microorganism in which deletion is caused to occur. Through PCR or a similar technique, there is selected, from the resultant antibiotic-resistant clones, a clone in which the plasmid has been inserted into the chromosome through recombination in a region homologous to the above-cloned region upstream or downstream of the target deletion region. The thus-obtained clone is repeatedly subcultured in a medium containing no antibiotic, to thereby select clones which have lost antibiotic resistance through removal of the plasmid from the chromosome by recombination between flanking homologous regions and through disappearance of the plasmid in bacterial growth. Through PCR or a similar technique, there can be selected, from the thus-obtained clones, a clone in which the target gene region has been deleted.

Suppression of expression of the gene of the present invention may be carried out through the so-called RNA interference method in which a short RNA fragment complementary to the 5'-end region of mRNA of the gene is synthesized, or a method in which a regulatory gene or a region for controlling expression of the gene of the present invention is disrupted or deleted. Particularly, modification of a region for controlling expression of the gene of the present invention is preferred. Specifically, the level of transcription of the gene of the present invention into mRNA can be increased or reduced by modifying the sequence of a promoter for controlling transcription of the gene.

No particular limitation is imposed on the microorganism into which the gene of the present invention is introduced or in which the gene is modified, and the microorganism may be, for example, a Gram-positive bacterium, a Gram-negative bacterium, or yeast. The microorganism employed is preferably a Gram-positive bacterium, particularly preferably, for example, a bacterium belonging to the genus *Lactobacillus* or *Bifidobacterium* which has been shown to be biologically safe. Among bacteria belonging to the genus *Lactobacillus,* bacteria of the *Lactobacillus casei* group, such as *Lactobacillus casei, Lactobacillus paracasei, Lactobacillus zeae,* and *Lactobacillus rhamnosus* are preferably employed, and *Lactobacillus casei* or *Lactobacillus rhamnosus* is particularly preferably employed. Examples of the microorganism originally having an oxygen-resistance-imparting gene include *Lactobacillus casei* YIT 9018 (FERM BP-665), *Lactobacillus casei* YIT 9029, *Lactobacillus casei* ATCC 334, and *Lactobacillus rhamnosus* ATCC 53103.

Bacteria belonging to the genus *Bifidobacterium,* which are obligate anaerobes, are labile to oxygen, low pH, or high acidity, and often encounter difficulty in handling (e.g., proliferation upon production, and survivability during storage). Since bacteria belonging to the genus *Bifidobacterium* exhibit physiological effects useful for humans, they have been applied to beverages, foods, or drugs by, for example, producing a mutant strain exhibiting oxygen resistance through improvement of breeding, or using an oxygen-impermeable container. However, the bacteria pose various problems, including difficult culturing, and a reduction in number of living cells during storage. Since bacteria belonging to the genus *Bifidobacterium* have been shown not to have the oxygen-resistance-imparting gene of the present invention, the bacteria are preferably employed as a microorganism of interest in which the gene of the present invention is introduced or modified.

The thus-obtained microorganism into which the gene of the present invention has been introduced or in which the gene has been modified can be employed for producing a food, beverage, or drug effectively exhibiting various physiological effects that are intrinsic to the microorganism, since oxygen resistance has been imparted to the microorganism or oxygen resistance thereof has been enhanced.

When the microorganism of the present invention in which the gene of the present invention has been introduced or modified is incorporated into a food or beverage or in a drug, living cells, heated cells (dead cells), or lyophilized cells of the microorganism may be employed. Alternatively, a cultured product containing the microorganism may be employed, or processed cells of the microorganism may be employed. Preferably, living cells of the microorganism are employed.

When the microorganism of the present invention is employed in a drug, the microorganism may be mixed with a solid or liquid pharmaceutical nontoxic carrier, and the mixture may be administered in the form of a conventional drug product. Examples of such a drug product include solid products such as tablet, granules, powder, and capsule; liquid products such as solution, suspension, and emulsion; and lyophilized products. Such a drug product may be prepared through a customary technique for drug production. Examples of the aforementioned pharmaceutical nontoxic carrier include glucose, lactose, sucrose, starch, mannitol, dextrin, fatty acid glyceride, polyethylene glycol, hydroxyethyl starch, ethylene glycol, polyoxyethylene sorbitan fatty acid ester, amino acid, gelatin, albumin, water, and saline. If necessary, the drug product may appropriately contain a conventional additive such as a stabilizer, a humectant, an emulsifier, a binder, an isotonizing agent, or an excipient.

The microorganism of the present invention in which the gene of the present invention has been introduced or modified may also be incorporated into a food or beverage in addition to the aforementioned drug product. When the microorganism is incorporated into a food or beverage, the microorganism may be employed as is, or mixed with various nutritional ingredients. The resultant food or beverage can be employed for producing a health food or food material effectively exhibiting various physiological effects that are intrinsic to the microorganism, since oxygen resistance has been imparted to the microorganism or oxygen resistance thereof has been enhanced. Specifically, when the microorganism obtained through the method of the present invention is incorporated into a food or beverage, the microorganism may be appropriately mixed with an additive which can be used in a food or beverage, and the mixture may be prepared, through conventional means, into a form suitable for edible use; for example, granules, particles, tablet, capsule, or paste. The microorganism may be added to a variety of foods; for example, processed meat products (e.g., ham and sausage), processed fish products (e.g., *kamaboko* and *chikuwa*), bread, confectionary, butter, and powdered milk. Alternatively, the microorganism may be added to beverages such as water, fruit juice, milk, refreshing beverages, and tea beverages. As used herein, the term "food or beverage" encompasses animal feeds.

Examples of the food or beverage of the present invention include fermented foods and beverages produced by use of the microorganism of the present invention, such as fermented milk, lactic acid baceteria beverages, fermented soybean milk, fermented fruit juice, and fermented plant extract. Such a fermented food or beverage may be produced through a customary method. For example, a fermented milk product may be produced through the following procedure. Firstly, only the microorganism of the present invention is inoculated into a sterilized milk medium, or the microorganism and another microorganism are simultaneously inoculated into the medium, followed by culturing, and the cultured product is homogenized to thereby yield a fermented milk base. Subsequently, a separately prepared syrup is added to and mixed with the fermented milk base, and the mixture is homogenized by means of, for example, a homogenizer, followed by addition of a flavor to the resultant mixture, to thereby yield a final product. The thus-produced fermented milk product may be in any form, such as a plain-type product containing no syrup (sweetener), a soft-type product, a fruit-flavor-type product, a solid product, or a liquid product.

The microorganism produced through the method of the present invention exhibits high oxygen resistance.
Therefore, when the microorganism is incorporated into a food or beverage product, since the microorganism exhibits high survivability therein, a reduction in number of living cells or an increase in rate of cell death is suppressed during storage of the product. In addition, the specification of the product is readily maintained, and the product effectively exhibits general physiological effects (e.g., regulation of intestinal functions) of a microorganism (e.g., a bacterium belonging to the genus *Lactobacillus*). When oxygen resistance is imparted, through the method of the present invention, to a bacterial strain belonging to the genus *Lactobacillus* or *Bifidobacterium* which originally has a specific physiological effect (e.g., anticancer effect or *Helicobacter pylori* eradication effect), or when oxygen resistance of the bacterial strain is enhanced through the method of the present invention, the bacterial strain can be applied to various foods and beverages, and the physiological effect of the bacterial strain can be enhanced by virtue of improvement of the survivability of the bacterial strain.

Hitherto, a container formed of an oxygen-impermeable packaging material (e.g., glass or aluminum-coated paper) has generally been used for storing a food or beverage product incorporating a bacterium belonging to the genus *Bifidobacterium* for the purpose of enhancing the survivability of the bacterium during storage of the product. In contrast, the bacterium belonging to the genus *Bifidobacterium* produced through the method of the present invention, which exhibits oxygen resistance, realizes employment, as a container material, of a resin having high oxygen permeability (e.g., polystyrene, polyethylene, or polyethylene terephthalate), since the bacterium exhibits high survivability and does not require strict anaerobic conditions. A container formed of such a resin is advantageous in that production cost can be reduced and the shape of the container can be changed freely, as compared with the case of a container formed of an oxygen-impermeable packaging material.

The gene of the present invention can also be employed for selecting, through screening, a microorganism exhibiting oxygen resistance.
Specifically, a microorganism exhibiting oxygen resistance can be selected through screening by determining the presence or absence of the gene of the present invention, and/or determining the level of expression of the gene.
For determination of the presence or absence of the gene and/or the level of expression of the gene, the presence or absence of a target gene in a microorganism, the number of copies of the gene, or the level of expression thereof is determined through southern hybridization, DNA microarray, or RT-PCR by use of a probe or primer which can detect the gene of the present invention or mRNA derived therefrom. A microorganism of interest is selected on the basis of the presence or absence of the target gene or the level of expression of the gene.

The recombinant vector of the present invention containing any of the polynucleotides shown in (d) to (f) or a portion (fragment) thereof can be obtained through a known technique (e.g., *in vitro* ligation) by use of any vector (e.g., pHY400, pSA1, or pYSSE3) having such a gene marker that can determine introduction of the vector into *Escherichia coli* and a microorganism of interest.

A host microorganism containing the aforementioned recombinant vector can be obtained through a known method. Specifically, when the recombinant vector is introduced into a host microorganism, electroporation or a similar technique may be employed. When the recombinant vector is integrated into the chromosome of the microorganism, there may be employed a method in which a recombinant vector having a DNA region homologous to that of the microorganism is introduced through electroporation or a similar technique, and then the vector integrated into the chromosome by homologous recombination is determined through, for example, PCR.

The DNA array or DNA chip of the present invention containing any of the polynucleotides shown in (d) to (f) or a portion (fragment) thereof can be prepared through a known technique such as photolithography. The DNA array or the DNA chip can be employed for selecting, through screening, a microorganism which expresses the gene of the present invention.

In order to effectively perform the aforementioned introduction of the gene of the present invention into a microorganism, modification of the gene, or screening of microorganisms, preferably, there is employed a recombinant vector containing the polynucleotide of the present invention or a portion thereof, a primer for PCR or RT-PCR containing a portion (fragment) of the polynucleotide of the present invention, a primer for PCR or RT-PCR which can amplify the polynucleotide of the present invention or a portion thereof, or a nucleic acid fragment for hybridization containing a polynucleotide which specifically hybridizes with the polynucleotide of the present invention or a portion of the polynucleotide.
The nucleic acid fragment (e.g., primer) which may be employed in the present invention is generally, for example, a nucleotide chemically synthesized on the basis of information on the nucleotide sequence of the gene of the present invention. Preferably, such a nucleotide has a partial nucleotide sequence corresponding to the nucleotide sequence of SEQ ID NO: 1 or 5, and includes 10 to 50 consecutive nucleotides (preferably 15 to 35 consecutive nucleotides).
The present invention will next be described in more detail by way of examples.

### Examples

### Example 1 Gene analysis of Lactobacillus casei YIT 9029 (gene extraction)

Genes of *Lactobacillus casei* YIT 9029 having high homology to those of thioredoxin reductase were retrieved from the relevant chromosomes based on, for example, the homology to known microorganism-derived thioredoxin reductase genes. Specifically, through the Lipman-Pearson method (Lipman, D. J. and Pearson, W. R., 1985, Rapid and sensitive protein similarity searches, Science 227: 1435-1441) by use of genetic information processing software (GENETYX, product of Genetyx Corporation), all the open reading frames (ORFs) possibly encoding proteins speculated from the genomic sequence of *Lactobacillus casei* YIT 9029 were subjected to homology analysis with respect to the amino acid sequences of the proteins encoded by the aforementioned respective genes. As a result, two ORFs having high homology to those of thioredoxin reductase were extracted from the genomic sequence. One of these two ORFs was found to correspond to the gene represented by SEQ ID NO: 1, and the gene exhibited 29.0% homology to the other gene (gene A) of *Lactobacillus casei* YIT 9029 having homology to thioredoxin reductase. However, the gene represented by SEQ ID NO: 1 was found not to have a CXXC motif (i.e., active center of thioredoxin reductase); i.e., the gene did not exhibit thioredoxin reductase activity. That is, the gene was found to be a gene whose functions have not yet been known. In contrast, gene A was found to have a CXXC motif, and thus considered as encoding thioredoxin reductase.

### Example 2 Isolation of gene-disrupted strain of Lactobacillus casei YIT 9029

A mutant strain in which the gene represented by SEQ ID NO: 1 (*fnr*) was deleted was prepared through the following procedure.
There were employed, as primers, an oligonucleotide 5'-cgggatccagatggctttttcacatt-3' (SEQ ID NO: 3), which had been designed by adding a sequence including a *BamH*I restriction site to the 5'-end of a sequence selected from the sequence of SEQ ID NO: 1, and an oligonucleotide 5'-aaactgcagccaccagtataccattacg-3' (SEQ ID NO: 4), which had been designed by adding a sequence including a *Pst*I restriction site to the 5'-end of a sequence selected from the sequence complementary to the sequence of SEQ ID NO: 1. By use of KOD Plus DNA polymerase (product of TOYOBO, product code: KOD-201) and according to an instruction attached to the enzyme, PCR was carried out with genome DNA of *Lactobacillus casei* YIT 9029 as a template. The thus-amplified DNA fragment is a partial sequence of the *fnr* gene lacking both the amino terminus and the carboxyl terminus. This product was mixed with an equiamount of Tris-EDTA (10 mM Tris (pH 8.0)-1 mM EDTA, hereinafter referred to as "TE") saturated phenol-chloroform-isoamyl alcohol (25 : 24 : 1). After thorough vortexing, the mixture was centrifuged at 15,000 x g for five minutes, to thereby separate it into two layers. The upper layer (aqueous layer) was recovered, and 3 M sodium acetate solution (pH 5.2) (1/10 amount to the aqueous layer) and 99.5% ethanol (thrice amount to the aqueous layer) were added thereto. The resultant mixture was allowed to stand at -20°C for 30 minutes or longer and then centrifuged at 4°C and 15,000 x g for 15 minutes. The supernatant was removed, and 70% ethanol was added to the precipitate for rinse. The thus-obtained mixture was centrifuged at 15,000 x g for five minutes. Thereafter, ethanol was removed, and the precipitate was dried under vacuum.

The precipitate was digested with restriction enzymes *BamH*I and *Pst*I (products of Takara Bio Inc.) at 37°C for 20 hours in K buffer (product of Takara Bio Inc.) reaction solution (100 µL). Subsequently, the aforementioned TE saturated phenol-chloroform-isoamyl alcohol treatment (mixing with solvent to recovery of aqueous layer) was carried out twice. An aqueous layer was recovered, and 3 M sodium acetate solution (pH 5.2) (1/10 amount to the aqueous layer) and 99.5% ethanol (thrice amount to the aqueous layer) were added thereto. The resultant mixture was allowed to stand at -20°C for 30 minutes or longer, and then centrifuged at 4°C and 15,000 x g for 15 minutes. The supernatant was removed, and 70% ethanol was added to the precipitate for rinse. The thus-obtained mixture was centrifuged at 15,000 x g for five minutes. Thereafter, ethanol was removed, and the precipitate was dried under vacuum.

There was employed, as a plasmid vector, pYSSE3 (Fig. 1), which has a replication region for *E*. *coli* originating from plasmid pUC19 and has an erythromycin-resistant gene (which functions both in *E. coli* and *Lactobacillus*) originating from plasmid pAMβ1. The pYSSE3 DNA was digested with restriction enzymes *BamH*I and *Pst*I (products of Takara Bio Inc.) at 37°C for 20 hours in K buffer (product of Takara Bio Inc.) reaction solution (100 µL). Subsequently, a 10-fold concentrated CIP buffer (product of TOYOBO) (20 µL) and water were added thereto so as to adjust the total volume to 200 µL, and calf intestine phosphatase (product of TOYOBO) (3 µL) was added thereto, followed by incubation at 37°C for two hours. Thereafter, the aforementioned TE saturated phenol-chloroform-isoamyl alcohol treatment and ethanol precipitation were carried out, and the precipitate was dried under vacuum.

The aforementioned DNA fragment consisting of an internal sequence of *fnr* and the plasmid vector which had been digested with the restriction enzymes were mixed each in an amount of about 0.01 to about 0.1 µg, and an equivolume of Solution I of DNA ligation kit Ver. 2.1 (product of Takara Bio Inc.) was added to the mixture, followed by incubation at 16°C for 30 minutes. Thereafter, the resultant product was placed on ice.

Next, the aforementioned reaction mixture (5 µL) was added to JM109 competent cells (product of TOYOBO) (100 µL), which had been placed on ice after dissolution, and the mixture was incubated for 30 minutes on ice after mild mixing. Thereafter, the reaction mixture was subjected to heat shock (42°C for 30 seconds), and then returned to ice. SOC medium (product of TOYOBO) (1 mL) was added to the cell liquid, and culturing was carried out at 37°C for one hour. The thus-cultured product was spread onto an LB agar medium (containing bacto-tryptone (10 g), bacto-yeast extract (5 g), sodium chloride (5 g), and agar (15 g) in 1 L) to which 500 µg/mL erythromycin (erythromycin for injection, product of Dainabot) had been added, followed by incubation at 37°C.

The thus-formed erythromycin-resistant colonies were grown in an LB medium to which 500 µg/mL erythromycin had been added, and recombinant plasmid DNA was extracted by means of Wizard Plus SV Minipreps DNA Purification System (product of Promega).

DNA transfer to *Lactobacillus casei* YIT 9029 was carried out through the following procedure. The relevant microorganism was grown in an MRS medium (product of Difco), and a culture liquid in a logarithmic growth phase was centrifuged at 5,000 x g and 4°C for five minutes, whereby cells were collected. The cells were washed once with ice-cooled 20 mM HEPES (pH 7.0) and once with 10% glycerol, and the washed cells were suspended in 10% glycerol (initial Klett value of culture liquid x 2 µL). The cell suspension (40 µL) and the recombinant plasmid DNA solution (2 µL) were mixed together, and the mixture was placed in a 2 mm-width cuvette for electroporation. Electroporation was carried out by means of Gene Pulser II (product of Bio-Rad Laboratories, Inc.) at 1.5 kV voltage, 200 Ω resistance, and 25 µF capacitance. An MRS medium (1 mL) was added to the thus-treated liquid, and the mixture was cultured at 37°C for one hour. Subsequently, the thus-cultured product was spread onto an MRS agar medium to which 20 µg/mL erythromycin had been added, followed by incubation under anaerobic conditions (provided by means of AnaeroPack Kenki (product of Mitsubishi Gas Chemical Company, Inc.)) at 37°C for two or three days.

A portion of the thus-grown erythromycin-resistant colonies was collected and suspended in TE (50 µL), and then the suspension was treated at 94°C for 2.5 minutes. A portion of the suspension was employed as a template for PCR. PCR analysis was carried out by using the following two primers: a primer selected from sequences located downstream of the *fnr* gene of *Lactobacillus casei* YIT 9029 chromosome; and a primer selected from sequences which are included in the plasmid vector and in the vicinity of a cloned *fnr* gene internal fragment. The PCR analysis revealed that the transferred plasmid was integrated into a region homologous to an *fnr* gene fragment included in the recombinant plasmid in the *Lactobacillus casei* YIT 9029 chromosomal *fnr* gene, whereby the *fnr* gene was divided (disrupted). The thus-obtained clone was employed as Δ*fnr*.

### Example 3 Gene analysis of Lactobacillus rhamnosus ATCC 53103 (gene extraction)

Genes of *Lactobacillus rhamnosus* ATCC 53103 having homology to the *Lactobacillus casei* YIT 9029 thioredoxin reductase gene (gene A) were retrieved in a manner similar to that described in Example 1. As a result, there were found a gene exhibiting 99.5% homology to gene A and considered as encoding thioredoxin reductase, as well as a gene exhibiting 29.3% homology to gene A. The latter gene exhibited 75.3% homology (in nucleotide sequence) and 89.0% homology (in amino acid sequence) to the aforementioned *fnr*. This gene was found to be a *Lactobacillus casei* YIT 9029 *fnr* gene ortholog, and a gene which, like the case of *fnr,* does not have a CXXC motif specific to thioredoxin reductase (i.e., considered as not having thioredoxin reductase activity), and whose functions have not yet been known.

### Example 4 Isolation of gene-disrupted strain of Lactobacillus rhamnosus ATCC 53103

A mutant strain in which the gene represented by SEQ ID NO: 5 *(fnr-r; Lactobacillus-rhamnosus-*derived *fnr* gene) (i.e., the function-unknown gene obtained in Example 3) was deleted was prepared in the same manner as described in Example 2, except that there were employed, as primers, an oligonucleotide 5'-TATGGATCCACACTAAAACGGTCAT-3' (SEQ ID NO: 7), which had been designed by adding a sequence including a *BamH*I restriction site to the 5'-end of a sequence selected from the sequence of SEQ ID NO: 5, and an oligonucleotide 5'-ATTCTGCAGTCGGTGCCTCACC-3' (SEQ ID NO: 8), which had been designed by adding a sequence including a *Pst*I restriction site to the 5'-end of a sequence selected from the sequence complementary to the sequence of SEQ ID NO: 5. PCR analysis revealed that the transferred plasmid was integrated into a region homologous to an *fnr-r* gene fragment included in the recombinant plasmid in the *Lactobacillus rhamnosus* ATCC 53103 chromosomal *fnr-r* gene, whereby the *fnr-r* gene was cleaved (disrupted). The thus-obtained clone was employed as *Δfnr-r.*

### Example 5 Growth of gene-disrupted strains of Lactobacillus casei YIT 9029 under aerobic conditions or anaerobic conditions

For investigation of the function of the *fnr* gene, there were carried out, in an MRS medium, anaerobic culture (dissolved oxygen concentration: low), aerobic static culture (dissolved oxygen concentration: moderate), and aerobic shaking culture (dissolved oxygen concentration: high) of cells of the *fnr* gene-disrupted strain obtained in Example 2, as well as cells of a strain in which a gene possibly responsible for oxygen resistance (NADH oxidase (*nox2*)*,* NADH peroxidase (*npr*)*,* thioredoxin (*trxA1*), or alkylhydroperoxide reductase (*ahpC*)) had been disrupted (i.e., Δ*nox2*, *Δnpr,* Δ*trxA1*, or Δ*ahpC*). Growth of each strains was determined and compared with that of wild-type *Lactobacillus casei* YIT 9029 strain. Each of the aforementioned oxygen-resistance-responsible-gene-disrupted strains was prepared in a manner similar to that described in Example 2.
Cells of the *fnr* gene-disrupted strain were precultured in an anaerobic MRS medium overnight, and cells of the other strains were precultured in an aerobic MRS medium overnight. Erythromycin was added to each of the culture media for the gene-disrupted strains other than the wild-type strain so as to attain a concentration of 20 µg/mL. Each of the thus-precultured products was inoculated (1%) into an MRS medium for main culture. For anaerobic culture, a culture medium through which nitrogen had been passed was added to a test tube; the gas phase of the test tube was replaced with nitrogen gas; and the test tube was sealed with a butyl rubber stopper, followed by static culture at 37°C. For aerobic static culture, a culture medium was added to a test tube, and the test tube was sealed with a silicon rubber stopper, followed by static culture at 37°C. For aerobic shaking culture, a culture medium was added to a test tube; the test tube was sealed with a silicon rubber stopper; and culturing was carried out at 37°C while the test tube was inclined and shaken at 160 rpm. In each of the aforementioned culture processes, turbidity was measured every two hours by means of a Klett meter (product of KLETT MFG).
As a result, all the mutant strains (including the *fnr* gene-disrupted strain) were well grown under anaerobic conditions as in the case of the wild-type strain. Under aerobic static culture conditions, the wild-type strain and the strains *Δnox2, Δnpr,* Δ*trxA1*, and Δ*ahpC* were well grown, but growth of the strain Δ*fnr* was considerably inhibited. Under aerobic shaking culture conditions, the wild-type strain and the strains Δ*nox2* and Δ*ahpC* were well grown, but growth of the strains Δ*npr* and Δ*trxA1* was slightly inhibited, and growth of the strain Δ*fnr* was considerably inhibited. Since growth of the strain Δ*fnr* was considerably inhibited even in the case where oxygen was present at a moderate level, the *fnr* gene was shown to be an oxygen-resistance-imparting gene. The above data revealed that the *fnr* gene greatly contributes to oxygen resistance, as compared with genes which have been conventionally known to be responsible for oxygen resistance, and that growth of the microorganism is prevented under aerobic conditions in the absence of the *fnr* gene.
The reason why cells of the *fnr* gene-disrupted strain were cultured in an anaerobic MRS medium for preparation of the precultured product is that the *fnr* gene-disrupted strain was not grown in an aerobic MRS medium. This fact also suggests that the *fnr* gene is essential for growth of the microorganism in the presence of oxygen.

### Example 6 Growth of fnr-r gene-disrupted strain of Lactobacillus rhamnosus ATCC 53103 under aerobic conditions or anaerobic conditions

Cells of the *fnr-r* gene-disrupted strain (*Δfnr-r*) prepared in Example 4 were spread with a platinum loop on an MRS agar plate medium to which erythromycin had been added so as to attain a concentration of 20 µg/mL. For aerobic culture, cells were incubated at 37°C for three days while the medium was allowed to stand still under non-controlled conditions. For anaerobic culture, cells were incubated at 37°C for three days under anaerobic conditions provided by means of AnaeroPack Kenki (product of Mitsubishi Gas Chemical Company, Inc.). As a result, the *fnr-r* gene-disrupted strain was grown on the MRS agar plate medium under anaerobic conditions, but no growth of the strain was observed under aerobic conditions. These data revealed that the *fnr-r* gene, which is an ortholog of the *Lactobacillus casei fnr* gene, is essential for growth of *Lactobacillus rhamnosus* ATCC 53103 under aerobic conditions, and the *fnr-r* gene is an oxygen-resistance-imparting gene.

## Claims

1. An oxygen-resistance-imparting gene encoding a protein selected from among the following proteins (a) to (c) :
(a) a protein having the amino acid sequence of SEQ ID NO: 2 or 6;
(b) a protein which has an amino acid sequence equivalent to the amino acid sequence of (a), except that one to several amino acid residues are deleted, substituted, or added, and which exhibits oxygen-resistance-imparting activity; and
(c) a protein which has an amino acid sequence having an identity of 85% or higher to the amino acid sequence of (a), and which exhibits oxygen-resistance-imparting activity.

2. An oxygen-resistance-imparting gene having a polynucleotide selected from among the following polynucleotides (d) to (f):
(d) a polynucleotide having the nucleotide sequence of SEQ ID NO: 1 or 5;
(e) a polynucleotide which hybridizes, under stringent conditions, with a polynucleotide having a nucleotide sequence complementary to the nucleotide sequence of (d), and which encodes a protein exhibiting oxygen-resistance-imparting activity; and
(f) a polynucleotide which has a nucleotide sequence having an identity of 75% or higher to the nucleotide sequence of (d), and which encodes a protein exhibiting oxygen-resistance-imparting activity.

3. A method for imparting oxygen resistance to or enhancing oxygen resistance in a microorganism, comprising introducing a gene as recited in claim 1 or 2 into the microorganism, or modifying the gene present in the microorganism.

4. The method according to claim 3, wherein the modifying the gene is enhancing expression of a gene as recited in claim 1 or 2.

5. A microorganism into which a gene as recited in claim 1 or 2 has been introduced or in which the gene has been modified.

6. The microorganism according to claim 5, which is a Gram-positive bacterium.

7. The microorganism according to claim 6, wherein the Gram-positive bacterium is a bacterium belonging to the genus *Lactobacillus.*

8. The microorganism according to claim 7, wherein the bacterium belonging to the genus *Lactobacillus* is *Lactobacillus casei* or *Lactobacillus rhamnosus.*

9. A food or beverage containing a microorganism as recited in any one of claims 5 to 8.

10. A drug containing a microorganism as recited in any one of claims 5 to 8.

11. A screening method for selecting a microorganism exhibiting oxygen resistance, comprising determining the presence or absence of a gene as recited in claim 1 or 2,
and/or determining the level of expression of the gene.

12. A recombinant vector containing a polynucleotide as recited in claim 2 or a portion thereof.

13. A host microorganism containing a recombinant vector as recited in claim 12.

14. A nucleic acid fragment which specifically hybridizes with a polynucleotide as recited in claim 2.

15. A DNA array or DNA chip containing a polynucleotide as recited in claim 2 or a portion thereof.
